# EUROPEAN PATENT APPLICATION

(11) **EP 4 684 794 A1**
(43) Date of publication of application: **28.01.2026**
(21) Application number: 24774080.6
(22) Date of filing: 18.03.2024
(51) Int. Cl.: A61K 36/82, A61K 33/14, A61P 29/00, A61P 15/08, A61P 35/00, A61P 21/00, A61P 31/04, A61P 31/12

(54) **TECHNICAL FORMULATION FOR ACTIVE ABSORPTION OF AMINO ACIDS BY HUMAN REPRODUCTIVE SYSTEM**

(30) Priority: 21.03.2023 CN 202310292958
(71) Applicant: Xu, Haohan, Shenzhen, Guangdong 518000 (CN)
(72) Inventor: Xu, Haohan, Shenzhen, Guangdong 518000 (CN)
(74) Representative: ZHAOffice SPRL
(86) International application number: PCT/CN2024/082258
(87) International publication number: WO 2024/193515

(57) **Abstract**

A technical formulation for the active absorption of amino acids by the human reproductive system,which technical formulation is prepared by physically extracting tea components and mixing same with sodium chloride. The technical formulation isbased on the regeneration and repair of the human nervous system and immune system,and is used in the treatment of various typesof inflammation,cancer,etc.

## Description

### Technical Field

The present invention belongs to the medical field and relates to a technical formulation for the active absorption of amino acids in the human reproductive system. The formulation involves regenerative medicine, sodium and chloride ion channels, changes in sodium ion concentration during biological competition in inflammation, cancer, and regeneration processes, and particularly relates to the application of this formulation and its derivatives in the preparation, prevention, and/or treatment of diseases.

### Background

Research indicates that humans have the potential for regenerative capabilities. Existing technologies targeting human regeneration are still in an imperfect stage. The technology of the present invention is based on the regeneration and repair of the human nervous and immune systems, enabling the regeneration and repair of muscles, blood vessels, bones, and other tissues and organs.

Current research in anti-aging and regenerative medicine involves numerous disciplines, with stem cell and gene editing technologies occupying a prominent position. For example, researchers at the University of Southern California used CRISPR stem cell therapy and gene editing technology in animal experiments to make lizards regenerate tails more perfectly, closer to the original appearance, including bones, nerves, and all other components. This is primarily because lizards are naturally regenerative animals, but their original regenerated tails differed from the original ones. Human intervention through gene editing has compensated for these defects.

Stem cell technology commonly uses mesenchymal stem cells derived from umbilical cords, placentas, adipose tissue, or bone marrow in clinical applications. These cells are cultured in cell factories and then injected for the repair of injuries to joints, hearts, ligaments, etc. Stem cell technology has already achieved the induction of cells from the earliest embryonic stages. For instance, a team of Chinese scientists led by Deng Hongkui used small chemical molecules to induce pluripotent stem cells, successfully reversing epidermal cells from adult mice into totipotent stem cells at the beginning of life. Stem cell technology involves cultivation in vitro, followed by injection into the human body, where they differentiate into pluripotent stem cells, which then become nerve cells, pancreatic islet cells, muscle cells, etc. Another example is the University of Tokyo and Stanford University, which developed a chemically defined and cytokine-free culture system to stably and long-term cultivate human-derived hematopoietic stem cells in the laboratory. Hematopoietic stem cells can differentiate into all types of blood cells and immune cells. The expansion of hematopoietic stem cells has always been a challenge, and this chemically defined culture system has clearly achieved a breakthrough.

Chemically induced stem cell technology is a highly significant regenerative technology. There are substantial differences between stem cell technology, gene editing technology, and the natural regeneration observed in salamanders, octopuses, and squids. The regeneration of octopus involves the complete restoration of entire tissues, such as limbs and their functions. Natural regeneration processes are more precise, devoid of fibrosis, and accompanied by minimal pain and inflammation over a short duration.

We believe two critical issues have been overlooked. First, changes in sodium ion concentration represent a method for stable regeneration in natural organisms and a means to avoid fibrosis. Second, regeneration is a repair process directed by the nervous system. For instance, our experiments have identified approximately a dozen fixed neural absorption pathways. Intervention with a solution can enhance functionality, while discontinuing the solution returns the system to its ordinary state. Conditions such as vitreous opacity, gum and periorbital muscles, neck flexibility, and sexual function can all be modulated by the solution.

For example, applying the formulation to eyes can repair functions of the eyes, brain, spine, and heart. Soaking the external genitalia with the solution can restore limb motor function and sexual function, encompassing a comprehensive combination of neural, muscular, and skeletal functions. Without the formulation, sexual function gradually reverts to its previous state within four months, and motor ability similarly declines. A reduction in neurotransmitters leads to instability, resulting in the gradual degeneration and regression of the human nervous system, which in turn causes vascular degradation, muscular atrophy, and slowed reactions. Of course, our research remains at the foundational stage, with numerous aspects requiring further refinement and study.

Accelerated regeneration and repair of the nervous system enable it to coordinate with the immune, circulatory, and muscular systems, collectively generating differentiated pluripotent stem cells at sites requiring repair to achieve precise regeneration. In anti-aging research, scientists have found that neurodegenerative diseases in the reproductive and central nervous systems are core factors driving human aging. For instance, degenerative conditions from the external genitalia to the sacral plexus persistently affect motor nerves in the lower limbs, such as the sciatic, common peroneal, and plantar nerves, further leading to degenerative diseases in the lower limb vascular system and bones. Similarly, degenerative conditions from the nipples to the brachial plexus and tibial nerves result in degenerative diseases in the neck and upper limbs. Analyzing methods to inhibit human aging reveals that supplementing key neural pathways-such as those from the eyes to the brain, external genitalia to the sacral plexus, and nipples to the cervical plexus-with this neurotransmitter is crucial. Maintaining and regenerating the nervous system from the perspectives of reproduction and vision is essential for suppressing human aging.

The present invention describes the unique role of the sacrum and sacral nerve plexus, which function like highly efficient "energy storage and charging" devices in the human body. Compared to the electrical signal intensity generated after dropping the solution to eyes, the signal from the sacral nerve plexus is dozens of times stronger. By soaking the external genitalia in the formulation solution, the human sacral nerve plexus can easily and continuously generate bioelectrical stimulation signals for approximately 24 hours. These signals propagate along fixed pathways, facilitating the repair and regeneration of numerous motor-related nerves in the body. In comparison, documented literature shows that spinal cord electrical stimulation therapy has been used clinically since 1967 and is currently practiced both domestically and internationally. On February 20, 2023, researchers at the University of Pittsburgh published a paper in Nature Medicine on spinal cord electrical stimulation aiding stroke recovery. Among the techniques discussed, epidural electrical stimulation is a clinically approved method. Clinical studies have found that external electrical stimulation of the cervical spinal cord or sacral nerve plexus improves chronic muscle weakness in post-stroke patients. In China, certain hospitals are also performing implantable sacral nerve stimulator surgeries, achieving cases where paraplegic patients regain the ability to walk. The key distinction of this technology is that it does not rely on external power sources, surgical procedures, or medications. Instead, it directly generates natural bioelectrical stimulation, offering a non-invasive medical approach to assist with movement disorders. Preliminary results have already been achieved in basic experiments focused on degenerative diseases.

In summary, while existing medical science and technology are highly advanced, there are still some limitations. Technologies such as active amino acid absorption through the eyes and external genitalia can serve as complementary approaches to modern human healthcare. Their promotion and application could alleviate patient suffering and achieve better therapeutic outcomes.

### Technical solution

To address the technical challenges of disease prevention and treatment in humans, particularly the issue of achieving regeneration, the present invention provides a formulation. The sacral region of the human body, specifically the sacrum and sacral nerve plexus, is an extremely critical core area for regeneration, possessing the functions of storing and continuously releasing electrical signals. The fixed pathway from the external genitalia to the sacral nerve plexus facilitates the regeneration and repair of the motor-related nervous system and delays aging. When the formulation solution is applied to the mucous membranes and skin of the external genitalia, such as through soaking for approximately half an hour, the sacral nerve plexus generates and transmits bioelectrical signals outward for about 24 hours. The bioelectrical signals transmitted by the sacral nerve plexus occur at intervals of approximately 0.6-1 second per cycle. For example, the formulation solution can be applied to the labia, clitoris, urethral opening, vaginal opening mucous membranes, and surrounding skin in females, or to the penis, glans penis, urethral opening, coronary sulcus mucous membranes, and surrounding skin in males. The bioelectrical signals are transmitted along fixed pathways from the sacral nerve plexus. Pathway 1: signals travel from the external genitalia (both male and female) to the sacral nerve plexus, then to the lumbar nerve plexus. Pathway 2: Signals travel from the sacral nerve plexus to the lumbar nerve plexus, then to the cervical nerve plexus, medulla oblongata, cerebellum, and the visual nerve area of the occipital bone, ultimately reaching the eyes. Pathway 3: Signals travel from the sacral nerve plexus and lumbar nerve plexus to the sciatic nerve, tibial nerve, and common peroneal nerve, then to the medial and lateral plantar nerve areas. Pathway 4: Signals travel from the sacral nerve plexus to the superior gluteal nerve and inferior gluteal nerve. Pathway 5: Signals travel from the sacral nerve plexus to the pudendal nerve, as well as nerves of the reproductive, urinary, and defecation systems, delivering bioelectrical stimulation signals. The bioelectrical signals from the sacral nerve plexus can block or replace inflammatory pain signals, alleviating pain in the spine and spinal cord, as well as pain in male and female reproductive organs and the male scrotum. They also inhibit motor neuron diseases, reduce inflammatory damage in the nervous system such as the spine, sciatic nerve, femoral nerve, common peroneal nerve, and superior gluteal nerve, and suppress inflammation in the female reproductive system while restoring female reproductive capacity. Additionally, these signals address issues such as erectile dysfunction and premature ejaculation, improve the quantity and quality of male sperm, enhance the rigidity of male reproductive organs, prolong the duration of pleasure, and restore sensitivity in reproductive organs. They also inhibit bacterial and viral diseases in reproductive organs, mitigate urinary system conditions like uremia and kidney inflammation, prevent kidney atrophy, and reduce inflammation in internal organs such as the liver, lungs, and pancreas. The sacral nerve plexus exhibits strong regenerative capabilities, facilitating the repair and regeneration of the entire human motor system, as well as the regeneration of internal organs such as the liver, pancreas, kidneys, and the urinary, reproductive, and defecation systems.

The present invention provides a formulation applied to the female reproductive system, breasts, and male nipple areas. By soaking or applying the formulation solution to the nipples for approximately half an hour daily, the nipple area actively absorbs the solution, and the resulting bioelectrical signals are transmitted along fixed pathways. Pathway 6: Bioelectrical signals travel from the nipples to the brachial nerve plexus and the tibial nerve plexus, then to the ulnar nerve, inhibiting inflammation and pain in the arms, chest, and back. Pathway 7: Signals travel from the nipples to the cervical nerve plexus and then to the facial nerves, suppressing lymphatic tumors around the neck; inhibiting facial sagging caused by the gradual weakening of muscles such as the platysma, sternocleidomastoid, trapezius, and masseter; alleviating mastitis, fibroadenomas, mammary hyperplasia, breast fluid accumulation, breast tumors and cancer, as well as pain in the breasts, neck, and arms. Breast lumps and hardness will soften, and breast fluid accumulation will rapidly decrease. The formulation also inhibits neck masses and tumors, prevents degeneration of neck muscles and sagging of facial muscles, and reduces the formation of neck wrinkles and wrinkles around the ears.

The invention further provides the use of the aforementioned formulation solution in the preparation of medicines for prevention and treatment.

The present invention is achieved through the following technical solution:
By increasing the concentration of the formulation solution and applying it to mucosal and skin areas, it can bind with the human nervous system, which actively absorbs the formulation solution.

Specifically, the formulation is either (a1) or (a2): (a1) A combination of sodium chloride and tea components, where the tea components are any combination of commonly used teas; (a2) A combination of sodium chloride and oolong tea (Tieguanyin).

Specifically, the tea components of the formulation are extracted by physical methods and mixed with sodium chloride. Teas can be classified in various ways, such as by fermentation degree. Teas are broadly categorized into six types: oolong tea (15%-30% fermentation), green tea (0% fermentation), black tea (100% fermentation), white tea (5%-10% fermentation), yellow tea (10%-20% fermentation), and red tea (70%-90% fermentation). The components of different teas are relatively similar, particularly the nearly 20 types of amino acids. In experiments, teas with longer fermentation times showed poorer efficacy, while oolong tea and green tea demonstrated better and more stable results. Over years of testing, Tieguanyin oolong tea has shown high stability. The therapeutic effects of tea lie in its nearly 500 components, including over 20 types of amino acids, which exist in stable proportions and concentrations. Consequently, after active absorption by the human nervous system, they can produce neurotransmitters with stable compositions and concentration ratios.

The tea components of the formulation are fully extracted through physical methods and mixed with sodium chloride to form a solution. The reason for using physical extraction is that the neurotransmitters of the human nervous system require natural products - long-term stable, side-effect-free, non-resistant, and consistent substances. This is because all regeneration methods in natural organisms involve no chemical drug components. Humans are learning from natural methods, and the exact composition, quantity, types, and proportions of human neurotransmitters remain inconclusive. Bioscientists, such as those cited in Yan Ning's course materials, propose that over 200 components of neurotransmitters have been identified so far, while course materials in Europe and America suggest that neurotransmitters may comprise nearly a thousand components. We propose a validation method based on experiments: to determine whether human neurotransmitters are correct, one can observe whether there is mucus secretion after eye drop application, whether fibrosis is inhibited, whether pain is rapidly suppressed, whether periorbital muscle growth occurs, and whether soaking damaged tissues leads to complete regeneration of muscles, blood vessels, nerves, and skin. Additionally, rapid repair and inflammation resolution in suppressing cancer and inflammation can be observed. If these characteristics are consistently observed, it can largely confirm that the requirements for complete human neurotransmitters are met. It is precisely based on such stringent conditions in eye drop experiments that we have currently identified plants corresponding to humans and turtles.

A technical formulation for the active absorption of amino acids by the human reproductive system. The formulation solution is applied to any part of the human body. By targeting the sodium ion channels and chloride ion channels in the human body, the formulation solution is actively absorbed by the nervous system and converted into stable human neurotransmitters, thereby enabling the regenerative and reparative capabilities of the nervous and immune systems. This, in turn, leads to the regeneration and repair of tissues and organs such as the circulatory system, muscles, and bones. Under relatively stable conditions of neurotransmitters in the human body, changes in sodium levels serve as a critical means of biological competition. For example, in the context of the sodium ion concentration in the formulation solution, the sodium ion concentration in the extracellular environment of malignant cancer cells is 451 mmol/L, while in inflammatory environments, it ranges from 200 to 300 mmol/L. In contrast, the sodium ion concentration outside the neural cell membranes of octopuses and squids is 440 mmol/L, which is an important reference value for animal regeneration. When the sodium ion concentration outside human neural cell membranes rises from 150 mmol/L to 4524 mmol/L and then returns to 150 mmol/L, the peak sodium ion concentration outside human neural cell membranes exceeds that of octopuses by approximately tenfold, that of malignant tumors by about tenfold, and that of inflammation by roughly twentyfold. The sodium concentration increases 30-fold from 150 mmol/L to 4524 mmol/L, significantly enhancing the intensity of bioelectrical stimulation signals. This, in turn, improves the human body's ability to suppress inflammation and cancer cells by 1 to 30 times. This spiral curve process facilitates the regeneration and repair of the nervous system. The nervous system has dozens of tentacle-like ducts inserted into the immune system, closely linking the two. Bioelectrical signals from the nervous system rapidly enter the immune system, increasing the bioelectrical signals received by the immune system by 1 to 30 times. As a result, the immune system's ability to secrete mucus is significantly enhanced, as is its capacity to produce immune cells. Consequently, the generation and maturation of immune cells within the immune system accelerate, and the speed of decomposing and eliminating inflammatory substances, as well as transporting them out of the body, is greatly improved. This enhances the body's ability to stop bleeding, debride wounds, and detoxify, while also inhibiting pain and itching, suppressing fibrosis of organs and tissues, and preventing tumors and vascular malformations. This formulation serves as a supplement for human amino acids and also as a supplement for human neurotransmitters.

The present application also provides an use of a technical formulation for active absorption of amino acids by the human reproductive system in the preparation of medicaments for preventing or treating human diseases. The drug efficacy comprises the content as described above. The dosage form of the drug is any one of sprays, injectable preparations, tablets, capsules, granules, suspensions, and pills, and further comprises pharmaceutically acceptable carriers and excipients; in practical applications, the methods of applying the formulation solution of the present invention to the entire human body include but are not limited to injections, topical application, instillation, nebulization, immersion, patches, etc. The finished products that can be produced include tablets, injections, nebulizations, plasters, patches, topical applications, acupoint patches, nebulized sprays, bottled liquids, capsules, and any other forms and combinations involved in pharmaceutical manufacturing processes.

### Embodiments of the Invention

In order to make the objectives, technical solutions, and advantages of the present invention clearer, the following embodiments are provided to further elaborate on the invention. It should be understood that the specific implementation cases described herein are only intended to illustrate the invention and do not limit its scope.

Throughout the specification, unless otherwise specifically indicated, the terms used herein are to be understood in their commonly accepted meanings in the field. Therefore, unless otherwise defined, all technical and scientific terms used herein have the same meanings as generally understood by those skilled in the art of the invention. In case of any discrepancy, this specification shall prevail.

Unless otherwise specifically stated, the various raw materials, equipment, and other items used in the present invention can be obtained through market purchase or prepared by existing methods.

The technical solution provided by the implementation of the present invention addresses the aforementioned technical issues, and the general approach is as follows:
Under relatively stable conditions of neurotransmitters in the human body, changes in sodium levels serve as a critical means of biological competition. Darwin believed that the evolutionary process of species is natural and undirected, while researchers suggest that changes in sodium ion concentrations can better explain this phenomenon. For instance, the sodium ion concentration outside the neural cell membranes of invertebrates such as octopuses is 440 mmol/L, whereas in felines, it is 200 mmol/L. In humans, the sodium ion concentration outside neural cell membranes is 150 mmol/L. Human muscles gradually degenerate without exercise, whereas feline muscles do not. Natural evolution has established specific sodium ion concentration values in the inherent nervous systems of different species. Microorganisms also utilize sodium ion concentrations to participate in the competition for survival of the fittest. When malignant tumors metastasize, the sodium ion concentration in the extracellular fluid of cancer cells is 451 mmol/L, while the normal sodium ion concentration outside human neural cell membranes is 150 mmol/L. The result of natural evolution is that cancer cells with a sodium ion concentration of 451 mmol/L dominate normal cells with a sodium ion concentration of 150 mmol/L. Similarly, when viruses or bacteria cause inflammation, inflammatory substances elevate the sodium ion concentration in the extracellular fluid to 200 - 300 mmol/L. The outcome of natural evolution is that inflammatory cells with a sodium ion concentration of 300 mmol/L dominate normal cells with a sodium ion concentration of 150 mmol/L. This leads to diseases such as cancer and tumors. Although the number of human cells is vastly larger than that of cancer cells, viruses, and bacteria, and the immune system can counterattack and eliminate these pathogens, in most cases, when malignant tumors begin to spread, the human immune system becomes suppressed, leading to failure and eventual death. Similarly, nervous system disorders such as pain and itching also follow the pattern of sodium ion dynamics. After applying the formulation, the sodium ion concentration outside human neural cell membranes rises from 150 mmol/L to 4524 mmol/L. This enables the suppression of malignant tumors with a sodium ion concentration of 451 mmol/L and the control of inflammation with sodium ion concentrations of 200 - 300 mmol/L. Consequently, it blocks the transmission of pain signals caused by malignant tumors and inflammation.

The future direction involves regenerative technologies gradually replacing organ transplantation. Human regeneration technology utilizing plant-based components is simple to administer-merely applying dropping the solution in eyes can promote the repair of the eyes, brain, spine, and heart, while soaking or applying the solution to the external genitalia can facilitate the continuous repair and regeneration of the sacral nerve plexus and motor-related nervous systems. This method offers flexibility in treatment timing, ease of use, and eliminates the need for immunosuppressive drugs. Such an approach could also serve regenerative medical purposes during interstellar travel. By cultivating tea plants on Mars or carrying tea and sodium salts during space missions, human regenerative medical technology could be readily implemented.

In the context of survival competition, species with regenerative capabilities, such as octopuses and squids, exhibit a sodium ion concentration of 440 mmol/L outside their neural cell membranes. In contrast, humans have a sodium ion concentration of 150 mmol/L outside their neural cell membranes. With the formulation solution, the sodium ion concentration outside human neural cell membranes can safely increase to 4524 mmol/L. Consequently, in survival competition, the 4524 mmol/L sodium ion concentration outside human neural cell membranes far exceeds the 451 mmol/L outside tumor cells, the 440 mmol/L outside octopus neural cell membranes, and the 300 mmol/L outside inflammatory cells. By adhering to the principles of natural evolution, humans, upon gaining regenerative capabilities, achieve a superior sodium ion concentration outside their neural cell membranes. This enables the suppression of cancer cell proliferation, viral and bacterial reproduction, cancer-related pain, and any other pain or inflammation.

Different animals' neurotransmitters correspond to distinct combinations of plant components and sodium salts. Through eye drop experiments, the growth or reduction of periorbital muscles in animals can preliminarily indicate the suitability of the neurotransmitter components. Subsequent regeneration experiments can further differentiate the specific neurotransmitters required for different animals.

### Experiments

The reason researchers conducted experiments directly on their own bodies is the significant difficulty of performing experiments on the human nervous system. During the foundational experimental phase from 2013 to 2016, we identified several fixed characteristics of the nervous system, such as fixed pathway transmission, pain suppression, and the relationship between mucus secretion and inflammation. These fixed traits were challenging to observe in animal experiments, as recording data on pathways, timing, locations, and quantities required human trials. For instance, it was impossible to observe the location of pain, the timing of its disappearance, or changes in intensity in animal experiments. Similarly, the secretion process of inflammation and mucus components, including their locations and timing, could not be adequately monitored in animal studies. This was particularly true for research involving the eyes, brain, and spine, where only direct participation by researchers could yield test data and materials, thereby truly verifying drug resistance and safety. Additionally, while there is extensive biological and medical research literature on tea components, experiments combining them with saturated sodium chloride solutions are relatively scarce. After a decade of self-testing by researchers, the formulation has been confirmed to be highly safe, with results far exceeding expectations. Dozens of relatives and friends were subsequently invited to participate in the experiments, and their outcomes were consistent with those of the researchers. Currently, the foundational research is in its concluding stage, gradually transitioning to pre-clinical observation and clinical phases.

### Experiments:

Safety and drug resistance testing: the formulation solution was applied daily via eye drops, nasal drops, oral administration, rectal clearance, and topical application to external genitalia, nipples, and skin. Each application lasted approximately 1 - 4 minutes. Participants typically underwent annual tests for liver and kidney function. There were no dietary restrictions, and consumption of high-purine late-night snacks, fried snacks, sugary beverages, alcohol, and starchy foods was allowed. Despite an increase in body weight from 80 kg to 95 kg, the participants' liver and kidney function indicators remained consistently within normal ranges: Albumin: 45.5 g/L; Gamma-glutamyl transferase: 40 IU/L; Bilirubin: 9.7 µ mol/L; Microglobulin: 1.66 mg/L; Creatinine: 70 µ mol/L; Serum urea: 3.68 mmol/L. Other participants who used this method for over six months also maintained normal test values.

Analysis: the sodium ion concentration exhibited a sustained and stable spiral curve, rising from 150 mmol/L to 4524 mmol/L and then returning to 150 mmol/L. The formulation solution was consistently absorbed by the human body. The metabolic rate of the participants was higher compared to the general population, leading to faster toxin elimination. Moreover, since the method involved non-invasive absorption through mucous membranes and skin-bypassing the digestive tract and injections-the impact on liver and kidney function was nearly negligible. The safety profile of this method surpasses that of oral medications, and drug resistance is virtually nonexistent.

### Eye-drop experiment:

Fixed Pathway: from the eyes to the occipital bone to the sacrum to the heart;
Fixed Characteristics: when applied 1-2 times daily, immediately after eye drops, a slight flowing sensation is felt in the meningeal lymphatic vessels of the occipital bone region, accompanied by a mild enhancement of cardiac signals. Almost simultaneously, continuous mucus secretion occurs from the posterior pharyngeal wall in the upward direction of the oral cavity. Mucus is instantly secreted from the meningeal lymphatic vessels in the left and right occipital regions and flows out. Inflammatory pain in the brain is relieved or disappears immediately after the eye drops.

Limit Test: the researcher applied the solution as eye drops every half-hour, exceeding 20 times a day. Signals were transmitted instantaneously in a point-to-point hopping manner-from the eyes to the occipital region, then to the sacrum, and further to the heart. This point-to-point transmission of electrical signals occurred at a rate of approximately 0.6 seconds per cycle and lasted for about 24 hours.

After the researchers administering eye drops more than 20 times a day for consecutive days and continuously for seven days, participants could feel the continuous point-to-point jumping method of transmission from the eyes to the occipital bone, from the occipital bone to the sacrum, and from the sacrum to the heart when their eyes were closed. The continuous strong signals had a significant impact on sleep, gradually leading to fatigue, weakness, and muscle soreness, but without other discomforts. Recovery could occur quickly after stopping the eye drops for three to four days. It is speculated that the concentration of sodium ions absorbed by the nervous system increases with the eye drops. Meanwhile, the rate at which sodium ions are metabolized is relatively slower than the absorption rate, leading to an excessive accumulation of sodium ions in the nervous system. Normal levels can be restored after three to four days of stopping the eye drops.

After extensive long-term eye drop testing, the most suitable application method has been confirmed: administering eye drops twice daily, for one minute each time. The drops are applied to the eyes and the upper and lower eyelids, as this pathway constitutes the body's most critical central neural pathway. After the electrical signals of the formulation are absorbed by the periorbital nerves, they travel instantaneously along the optic nerve tract to the brain, spine, and heart regions. Daily eye drop application consistently influences the eyes, brain, spine, and heart, thereby inhibiting degenerative eye diseases, degenerative brain diseases, degenerative spinal diseases, and degenerative heart diseases. It also suppresses pain and inflammation while repairing and regenerating damage to the eyes, brain, spine, and heart.

The researcher had suffered from persistent headaches for many years, with intense, bell-ringing-type headaches occurring two to three times per month in 2012. Medical examinations revealed lacunar cerebral infarctions with two defect holes in the left and right brain. After using the eye drops, follow-up brain CT scans showed that the holes in the left and right brain had been regenerated and repaired. Chronic migraines and cluster headaches completely disappeared.

Multiple middle-aged and elderly users who applied the formulation solution as eye drops experienced growth in periorbital muscles, including the orbicularis oculi, temporalis, and zygomaticus muscles. Sunken eyeballs gradually returned to their original positions, periorbital wrinkles reduced, and ocular injuries and diseases were regenerated and repaired. Examples include the resolution of cataracts, repair of retinal detachment, and improvement of vitreous opacity. Visual acuity remained well-preserved, cardiac function improved, and sleep quality enhanced.

### Animal Eye Drop Experiments:

Researchers applied the formulation solution originally developed for human use to animal experiments, administering it as eye drops on the animal ocular mucosa 3 - 10 times daily. The results were as follows: frogs exhibited an intoxicated, poisoned state after the eye drops; turtles subjected to continuous eye drops for one week developed sunken periorbital muscles; Java sparrows showed immediately pronounced heart palpitations after administration and died within days; hamsters died after several applications, and rabbits died after a few days of treatment. Prolonged eye drop experiments caused damage to or death of the animals' central nervous systems.

Subsequently, the formulation for the eye drop experiments was modified to a combination of longan leaf and sodium chloride. When tested on turtles, the eye drops led to growth in the periorbital muscles, and the turtles achieved regenerative repair of severed tails. The methodology involved: cutting the turtle's tail at a point two centimeters from the tip, soaking the wound in the formulation solution for one to two hours or more. Within two to three days, a black scab formed on the tail. The solution was applied topically around the tail two to four times daily, avoiding direct contact with the black scab. The turtles were also fed the formulation solution two to three times daily, with each session lasting five minutes, during which the upper half of the turtle's body was immersed in the solution while the tail remained dry. Throughout the regeneration experiment, the tail was kept from contact with water. The scab thickened over about a week, with white hairs appearing around and on top of the scab. The black scab continued to rise, and missing muscle and bone tissues gradually regenerated. Subsequently, white hairs reappeared, the black scab further elevated, and partial regeneration of the tail's muscle and bone tissues continued, with no scar fibrosis observed during the process. The regeneration speed was relatively slow: the thicker part of the tail took about two months to heal, while the thinner part grew only about three millimeters over three months. Crucially, the thick scab persisted and advanced slowly.

When the longan leaf and sodium chloride formulation was tested on the ocular mucosa of other animals, frogs again exhibited intoxicated and poisoned symptoms. Frogs, Java sparrows, hamsters, and rabbits all died within days, with their central nervous systems similarly damaged, leading to death.

Based on the eye drop experimental results, the researchers proposed a hypothesis: neurotransmitter isolation exists between animal species, regenerative capacity is controlled by the nervous system, and animal regeneration is regulated by changes in neurotransmitter concentration and sodium ion concentration. Different animal neurotransmitters correspond to distinct plant components and sodium salt combinations.

According to the eye drop experimental results, using fruit flies and mice to study human nervous system drugs carries significant risks and is flawed. The human nervous system, particularly the central nervous system, requires stable and precise neurotransmitter components. Instability in these components is a critical factor contributing to heart and brain diseases, including psychological disorders such as Alzheimer's disease and depression. This also explains why animal experiments on mice and monkeys often yield promising data, while human trials frequently fail.

### External genitalia experiment:

Fixed pathway: From the external genitalia to the sacral plexus, then transmitted through several paths;

From the sacral nerve plexus to the lumbar plexus, then to the occipital bone and the eyes; from the sacral nerve plexus to the lumbar plexus, then to the sciatic nerve, common peroneal nerve, and tibial nerve, reaching the plantar nerves; from the sacral nerve plexus to the superior gluteal nerve and inferior gluteal nerve; from the sacral nerves to the pudendal nerve, femoral nerve, dorsal nerve of the penis, perineal nerve, anal nerve, and others. The sacral nerve plexus transmits signals directly to the lumbar plexus. Compared to the intensity of eye drops, the signals here are stronger, occurring approximately every 0.6 seconds. While extreme eye drop experiments may disrupt sleep due to electrical signals, experiments with external genitalia solutions do not affect sleep.

Fixed Characteristics: soaking or topical application is relatively comfortable with minimal irritation. The sacral nerve plexus, lumbar nerve plexus, and related nerves undergo regeneration and repair. Elderly individuals with cold lower limbs experience rapid warming of the legs, disappearance of limb stiffness, and restored flexibility in the hips, abdomen, and lower limbs. Walking and motor abilities significantly improve, with gradual increases in gluteal and leg muscle mass. Sexual and reproductive functions improve, including increased sperm volume, enhanced rigidity, and prolonged endurance in males. Pain relief is notable, with conditions such as sciatica, lumbar disc pain, and seminal vesicle pain alleviated or eliminated. After continuous soaking for two weeks or more, if symptoms resolve, the application time should be reduced to one minute daily. Prolonged use exceeding 30 minutes daily for more than two weeks may lead to sodium ion metabolism issues, causing nervous system fatigue. Discontinuation for three to four days allows for recovery.

### Nipple Experiment:

Fixed pathways: from the nipple to the cervical plexus to the facial nerve; from the nipple to the cervical plexus and brachial plexus to the radial and ulnar nerves.

Fixed characteristics: Immersion or application is relatively comfortable, with some participants experiencing mild nerve pulsations in the neck and cervical region. The neck and cervical region regain flexibility from stiffness, approaching the condition of youth. When combined with eye drops, it can promote fuller breast growth in women, with an increase in cup size. Pain such as neck rotation pain, arm pain, scapular pain, and shoulder or elbow pain is quickly alleviated and eliminated. Breast inflammation and fluid accumulation rapidly decrease. Breast lumps and hard masses gradually soften. Daily immersion of the nipples for 30 minutes to one hour over four to eight weeks breaks down hard masses into smaller pieces. Lymph node masses and hard lumps in the neck and cervical region soften and shrink. Wrinkles around the face and ears reduce, and muscles gradually increase. Neck wrinkles decrease, and muscles gradually increase.

Analysis of external genital and breast experiments shows high comfort levels, with significant breast-enlarging effects for women with poor breast development and noticeable cup size growth. There is a rapid regenerative and reparative ability for motor impairments of the limbs and spine. However, with daily electrical signal intensity of over 30 minutes, it is advisable to rest for a few days every two weeks to allow the electrical signal intensity to decrease. Otherwise, it may easily lead to fatigue. The analysis suggests that the nervous system's rapid absorption of sodium ions exceeds the excretion rate, and accumulation over several weeks may cause metabolic issues in the nervous system. Stopping for three to four days restores normal function. After symptoms disappear, daily immersion for just one minute is sufficient to maintain the regenerative and reparative function of the sodium ion spiral curve, ensuring long-term health.

Other Fixed Pathways: In basic experiments, the pathways through which the human nervous system actively absorbs the formula solution have been summarized as follows:
Fixed pathway of the lung experiment: from the mouth and nose to the lungs, to the ears, neck, and spine, and to the sacrum of the spine.

Fixed characteristics: The process is comfortable, using the method of inhaling through the mouth and exhaling through the nose. Most users begin to cough and expel phlegm after about 7 minutes of nebulization. After the nervous receptors in the lungs, such as stretch receptors, ciliary receptors, and cough receptors, actively absorb the components of the formula, they rapidly convert into neurotransmitters. The nervous and immune systems of the lungs work together to secrete mucus, which is transported to the throat and actively expelled through coughing. With 30 minutes of nebulization, this method can expel approximately 60 ml of mucus and can be performed multiple times daily to continuously remove inflammatory fluid from the lungs, inhibit the proliferation of bacteria and viruses in the lungs, suppress pulmonary fibrosis, and gradually soften and eliminate lung masses. It also alleviates pain in the neck, chest, and back, and inhibits lung bacteria and viruses.

In basic experiments, researchers conducted comparative nebulization tests using alternative components such as Isatis root, Forsythia, Houttuynia cordata, and sodium chloride. These quickly triggered inflammation and pain in the neck area, as well as inflammatory reactions in the sacrum. In contrast, using the formula components for nebulization over five to ten days gradually cleared the side effects caused by nebulization with Isatis root and other alternatives.

Analysis: After nebulization with saturated sodium chloride and tea components, the absorption by nervous receptors in the lungs converts them into neurotransmitters, which can then be transmitted to the spine. Using other plant components with saturated sodium chloride for lung nebulization leads to inflammatory reactions and issues such as neck pain, due to problems with the resulting neurotransmitters.

The fixed pathway of the nasal experiment is from the left and right nasal sides to the trigeminal nerve and then to the area behind the ears, and from the nasal sides to the forehead.

Fixed characteristics: The solution is dripped onto the left and right nasal sides, approximately 1 cm away from the nostril exit, near the intersection of the nasolabial folds and the nasal wings. Mucus secretion occurs inside the nasal cavity, starting about 1 minute after application, and it suppresses nasal pain and trigeminal nerve pain. The linear transmission runs from the nasal sides to the trigeminal nerve and then to the area behind the ears. Some elderly users also report the solution reaching the forehead after nasal application.

The fixed pathway of the oral experiment is from the mouth, tongue, and teeth to the neck and throat.

Fixed characteristics: 10-20 ml of the solution is poured into the mouth, held for 2-5 minutes, and then spit out. The mouth feels numb, and phlegm is expelled from the throat. The teeth send signals to the alveolar bone, and electrical signals are transmitted downward through the neck. The sensation is strong when inflammation is present and weak or absent when inflammation is mild or absent. Gargling 1-2 times daily results in loose teeth becoming firm and the gum muscles thickening and increasing.

The fixed pathway of the rectal experiment is from the rectum and bladder to the sacral nerve plexus of the spine.

Fixed characteristics: 10 ml of the solution is introduced into the rectum, and after waiting for one minute, significant gas, bowel movements, and urination occur. Mucus is secreted each time, and occasionally, transparent, foul-smelling mucus is expelled from the rectum. The area of exertion during bowel movements is clearly the sacral and lumbar regions. Before using the formula, constipation required full-body exertion, including the neck and entire spine, with the face turning red. However, after using the formula solution, exertion is limited to the sacral and lumbar regions, with no strain in the head or facial redness.

Analysis: The first rectal experiment is comfortable, but the comfort decreases with the second consecutive session. Continuing beyond three sessions is difficult due to strong bowel movement signals causing significant rectal discomfort. The signals transmitted from the rectum to the sacral nerve plexus are confined to the rectal and sacral areas, with limited outward transmission. In contrast, the electrical signals transmitted from the external genitalia to the sacral nerve plexus are not only prolonged but also of higher intensity. The sacral region and sacral nerve plexus's ability to store and transmit electrical signals over an extended period is crucial for regenerating and repairing spinal injuries and restoring limb motor function. Spinal cord injuries, high-level paralysis, incontinence, and chronic pain are common challenges faced by many spinal patients. Alleviating pain in paralyzed patients, improving neurological function, and restoring limb motor function are clinical difficulties. Spinal cord injuries can also lead to other issues, such as lung infections, intestinal obstructions, muscle atrophy, fractures, sexual dysfunction, and deep vein thrombosis in the lower limbs. Immersing the external genitalia can easily achieve sustained bioelectrical signals in the sacral nerve plexus.

### Comparison of Eye Drop Experiments and External Genitalia Experiments:

Fixed Pathways: The biological signal transmission pathway in the eye drop experiment is from the eyes to the occipital bone, to the sacrum, and to the heart. In the experiment where the liquid is applied to the external genitalia, the biological signal transmission pathway is from the external genitalia to the sacral nerve plexus, to the lumbar nerve plexus, to the medulla oblongata, to the cerebellum, to the occipital region, and to the eyes.

### Fixed Characteristics:

Researchers discontinued the one-minute daily eye drop experiment with the formula solution for two months, using only the one-minute daily immersion of the external genitalia in the formula solution. Biological signals were transmitted from the external genitalia to the sacral nerve plexus, to the medulla oblongata, cerebellum, occipital region, and eyes. After one week, symptoms of visual impairment, such as floaters in the eyes, gradually appeared. Prior to the external genitalia immersion experiment, if the daily one-minute eye drop experiment with the formula solution was discontinued, inflammatory issues would arise in the meningeal lymphatic vessels of the occipital region, accompanied by slight swelling and elevation of the occipital area. After resuming the eye drops, the inflammatory substances would clear, and the occipital region would return to normal. However, during the two-month period when the eye drop experiment was discontinued and only the one-minute daily external genitalia immersion was performed, no inflammatory reactions occurred in the meningeal lymphatic vessels of the occipital region. This suggests that the inflammatory substances in the meningeal lymphatic vessels can be regulated in two ways: one is through excretion via the anterior and posterior pharyngeal walls during the eye drop experiment with the formula solution, and the other is through excretion via the neck lymph nodes after immersion of the external genitalia in the formula solution. It is hypothesized that the fixed pathway from the sacral nerve plexus to the eyes repairs the entire body's motor system and limb flexibility, including the nerves associated with movement in the eyes, visual areas, cerebellum, medulla oblongata, lumbar nerve plexus, and other motor-related nerves.

After resuming the eye drop experiment with the formula solution, visual issues such as floaters were quickly resolved. The fixed pathway of the eye drop experiment primarily targets the eyes, brain, spine, and heart, whereas the sacral nerve plexus is more involved in functions related to reproduction and movement, such as the lumbar plexus, cerebellum, medulla oblongata, partial brain functions, partial eye functions, hip nerves and muscles, sciatic nerve, sexual function, urinary function, liver function, etc.

Analysis: The human nervous system is highly complex, often exhibiting overlapping and collaborative functions, but each nervous system's fixed pathway tends to have distinct inclinations.

For example: The use of the formula solution as eye drops has more pronounced effects on the eyes, brain, certain spinal diseases, and heart conditions.

Immersion of the external genitalia in the formula solution has more significant effects on the regeneration and repair of nerves and muscles related to movement in the pelvis, thighs, hips, and spine, as well as on the regeneration and repair of sexual organ function.

Immersion of the nipples in the formula solution has more notable effects on the regeneration and repair of pathways from the nipples to the mammary glands, neck, and face, as well as the nerves and muscles of the upper limbs.

Other applications, such as nasal drops with the formula solution, have more evident effects on the pathway from the nasal nerves to the trigeminal nerve and ears.

Oral immersion with the formula solution has more pronounced effects on reducing inflammation from the mouth to the neck, as well as on strengthening gum muscles and teeth.

Rectal application of the formula solution has more significant effects on promoting gas, urination, and excretion, suppressing inflammation in the perianal area, inhibiting rectal polyps, and supplementing neurotransmitters in the digestive system.

### From a Time Perspective Analysis:

For the general population, applying the formula solution to the aforementioned organs and tissues for 2 - 5 minutes twice daily, in the morning and evening, is a relatively simple method to maintain health effects. Daily use is not strictly mandatory. If the use of the formula solution is discontinued, there are no associated risks. For severe masses, such as those in the liver, lungs, or breasts, using the solution for one to two hours daily until the masses shrink or disappear is recommended.

Therefore, the duration of formula solution usage can be flexibly adjusted, determined by a physician or individually based on specific circumstances.

### Effects of Immersing External Genitalia in the Formula Solution on Internal Organs:

Fixed Pathway: Biological signals travel from the external genitalia to the sacral nerve plexus, then to the lumbar nerve plexus, the nerves of the urinary and reproductive systems, and finally to internal organs such as the kidneys and liver.

### Fixed Characteristics:

Regarding the elimination of internal organ inflammation or severe tumors, our view is that the aforementioned methods should focus on intestinal clearance, oral immersion, and external genitalia immersion. Applying the formula solution to the external genitalia and surrounding areas for 2 - 5 minutes twice daily, in the morning and evening, is an effective way to maintain optimal results. For patients with kidney disease, immersing the external genitalia twice daily for approximately 30 minutes each time, continuing for one week followed by a one-week break, and repeating this cycle is advised. Generally, 4 - 8 weeks can be considered as one treatment course, continuing until urination normalizes and medical tests indicate normal kidney function.

### Pharmacokinetic Analysis:

The absorption of the formula solution by the human body follows certain fixed pathways of the nervous system. The metabolism of inflammatory substances and drug components immediately manifests as mucus secretion, which is then expelled by the immune and digestive systems. The entire process is precise and controllable, without affecting normal sleep and diet. The bioelectrical signals rapidly inhibit pain, significantly alleviating the suffering of critically ill patients. This method offers advantages such as being non-invasive, having a comfortable first-pass effect, and good patient compliance. It reduces adverse reactions and avoids the burden of gastrointestinal and hepatic stimulation. The human body exhibits good tolerance to this approach.

Route of Administration: Absorption through the skin and mucous membranes via the nervous system; microinjections into the subcutaneous layer.

Administration Time: Application to mucosal areas for 2 - 5 minutes daily.

Single Dosage: Eyes: Approximately 0.1 - 0.3 ml; Nasal sides: Approximately 0.1 - 0.7 ml; Oral cavity: 20 - 30 ml; Rectum: 10 ml; Lungs: 5 ml; Nipples: 10 - 20 ml; External genitalia: Approximately 20 ml; Skin application: No dosage restriction.

The following will provide a detailed explanation with reference to several embodiments.

### Example

This example uses tea leaves (purchased online) and pure sodium chloride (purchased online). The tools used include a pressure cooker, a white porcelain bowl, and a white porcelain spoon. A regeneration experiment was conducted on a muscle-deficient area, specifically including the following steps: preparation of the mixed solution: add 1000 ml of purified water to a 304 stainless steel pressure cooker; add 200 grams of Tieguanyin oolong tea leaves; heat for 30 minutes; wait for 2 hours, then open the lid and transfer the tea liquid into a porcelain bowl; add 400 grams of sodium chloride and stir with a white porcelain spoon; during the addition of sodium chloride and after the mixed solution is formed, avoid contact with any metal or plant components. Excess salt may remain at the bottom.

### Regeneration of skin, muscles, blood vessels, and nerves in a decayed cavity on the calf:

A researcher accidentally sustained a deep cut to the calf muscle. In an attempt to disrupt wound healing, the researcher applied sodium chloride granules to the wound for several days, causing the wound to expand and become infected with pus. The researcher then treated the wound with a mixture of iodine and phenol solution, which worsened the infection, resulting in a cavity-like wound measuring approximately 2 × 3 cm in area and 1 cm in depth, with decayed tissue.

Subsequently, the researcher used a high-concentration formula solution to soak the 2 - 3 cm² wound. The solution was replaced hourly, and this was repeated over ten times. The pus in the wound rapidly decomposed and detached on its own. Within ten hours, a black scab formed. Over the next two months, to keep the area above the scab dry, the formula solution was applied around the scab within a 1 cm perimeter, approximately three times daily.

Three days later, a translucent scab formed around the black scab. After seven days, a brownish mucus secreted continuously beneath the translucent scab, quickly solidifying and elevating the black scab. In the sixth week, the researcher opened the scab and observed the muscle regeneration process: beneath the scab was a regenerative fluid, on which floated more than ten small, lotus-leaf-like white adipose stem cells. After photographing the findings, the researcher cleaned away the regenerative solution and white cells, then reapplied the formula solution to soak the area. The scab reformed and naturally fell off after eight weeks. The regenerated muscle and skin were complete, smooth, and seamlessly integrated, with the skin texture gradually improving over time.

### Example

A 74-year-old female patient, weighing 40 kg, suffered from lumbar disc herniation, calf cramps, and unsteady standing. In August 2021, a lumbar CT scan revealed the following: L5-S1 disc herniation to the right posterior, compression of the right intervertebral foramen, density changes and calcification in the L4-L5 facet joints, and nerve compression. The patient daily used the formula solution for eye drops, intestinal clearance, and microinjections. By September 2022, a follow-up lumbar CT scan showed only disc bulges at L3-L4 and L4-L5. After several weeks of immersing the external genitalia in the formula solution, the patient experienced noticeable warmth in the lower limbs, improved walking strength, enhanced balance, restored steady gait, and alleviation of calf cramps.

A 55-year-old female patient was diagnosed with vitreous opacity, a 3-4 PD-sized retinal hole, peripheral retinal detachment around the hole, old laser spots in the periphery, retinal detachment, and a macular hole in the macular area. After six months of participating in the eye drop experiment, an ophthalmological examination revealed round optic discs with clear boundaries in both eyes, clear foveal reflexes in the maculae, and no abnormalities on the remaining retinal surfaces. The retinal detachment had resolved, and the patient was diagnosed with a left macular hole without traction abnormalities. The macular hole showed no upward protrusion, the bulge had flattened, ocular edema was absent, traction was within normal limits, visual distortion had disappeared, and visual acuity had returned to normal, eliminating the need for surgery.

A 50-year-old male patient suffered from kidney stones, nephritis, hepatitis, and palmar spotted spider nevi. After six months of using the formula solution for intestinal clearance and external genitalia immersion, the patient experienced reduced and eventually eliminated kidney pain, gradual reduction and disappearance of kidney stones, and normalized liver and kidney function test results.

A 60-year-old male patient experienced weak urination, reduced sexual function, and multiple inflammatory conditions of the genitalia. After several weeks of immersing the external genitalia in the formula solution, the patient reported stronger urination, resolved inflammation, and restored sexual function.

A 37-year-old male patient was diagnosed with lacunar cerebral infarction, migraines, and cerebral vascular stenosis. After one year of using the formula solution as eye drops, the migraines quickly resolved after application, and the cerebral infarction site disappeared.

### Hemostasis and Debridement:

In multiple tests, researchers found that immersing injured areas in the formula solution quickly stopped bleeding, provided rapid relief from itching and pain, and achieved debridement effects without the need for manual intervention.

### Experimental Analysis:

It is hypothesized that the root cause of aging lies in the degeneration of the reproductive nervous system, which accelerates overall aging. Therefore, immersing the external genitalia in the formula solution and administering eye drops are critical anti-aging methods. Replenishing neurotransmitters at the source is key to both anti-aging and regenerative repair. All observed cases demonstrate restoration to a state close to youth, with no instances of malformation or unexpected outcomes.

In On the Origin of Species, Darwin emphasized that embryos are key to the diversity of animals. Embryos develop in an orderly sequence: the nervous system appears first, followed by the heart and blood vessels, and then other organs and tissues. After absorbing the formula solution, the nervous system achieves a perfectly controlled regenerative capacity. The nervous system first suppresses pain, directs the immune system to eliminate inflammation, and then enables the regeneration and repair of muscles, blood vessels, bones, and other tissues at the required sites. Within the nervous system, the reproductive system and the eye-brain central system are of particular importance.

Through experiments, we have observed that this solution, containing sodium and plant components, aligns well with the neurotransmitter requirements of the human nervous system. Neural excitation propagates between neurons as electrical signals, while chemical changes occur at the points where two neurons connect. In the process from the "presynaptic membrane - synaptic cleft - postsynaptic membrane," there is a transformation of "electrical signals - chemical signals - electrical signals." Neurotransmitters are chemicals released by nerve endings that transmit information between neurons or between neurons and effector cells.

Traditional medicine classifies neurotransmitters into six major categories: choline, monoamines, amino acids, gases, neuropeptides, and purines. Neurobiologists, such as Yan Ning, suggest there are approximately 200 types of neurotransmitters, while some scientists propose the number could be as high as thousands. Thus, the specific components of human neurotransmitters and their proportional relationships require further in-depth research.

Through the gradual accumulation of successful plant-based regeneration experiments in humans, it can be inferred that tea components contain the most suitable composition and proportional relationships for human neurotransmitters.

Tea contains over twenty types of amino acids, with their proportions and respective percentages being highly stable. Additionally, the approximately 500 trace substances in tea, such as vitamins, coenzymes, and inorganic small molecules, align well with the requirements of human neurotransmitters.

Thus, through extensive eye drop experiments, a relatively accurate conclusion can be drawn regarding the types and proportional relationships of human neurotransmitters: human neurotransmitters correspond to a combination of sodium salts and tea components.

### Tea Research Data:

In 2020, a research team from the Zhejiang Provincial Center for Disease Control and Prevention selected green tea, Tieguanyin, and Dahongpao, each weighing 2 grams, and steeped them in 50 ml of hot water (measured at 90° C) for 30 minutes. At this point, the concentration was recorded as 40 mg/ml (typical for the first brew of tea). After cooling to room temperature, the tea was diluted with cell maintenance medium to various experimental concentrations. The experiment involved in vitro virus inactivation tests using tea solutions at different concentrations (2.5-10 mg/ml), with boiled water and commercially available drinking water set as control groups. After treating 100 TCID50 of the novel coronavirus with different concentrations of tea solutions and control groups for one hour, the mixtures were used to infect Vero cells and cultured for 48 hours to measure the viral nucleic acid proliferation levels in each group.

The results showed that compared to the untreated virus control group, the nucleic acid proliferation of the novel coronavirus treated with tea solutions decreased by 10,000 to 100,000 times. In contrast, the results for the boiled water and drinking water control groups were consistent with the virus control group, indicating that tea solutions have a strong extracellular neutralizing effect on the novel coronavirus, with the inhibitory effect increasing as the tea concentration rises.

In 2022, Professor Wu Yonglin from Tianjin University published a paper exploring the use of Chinese tea components as raw materials to extract natural tea nanodots (TNDs). The study confirmed the antibacterial mechanism of TNDs against methicillin-resistant Staphylococcus aureus and their efficacy against the H1N1 influenza virus. It demonstrated that combining TNDs with luteolin for nebulized inhalation is a safe and effective treatment for mixed bacterial and viral pneumonia.

Tea polyphenols represent the first botanical drug approved by the U.S. FDA under its Botanical Drug Guidance. Marketed under the trade name Veregen, it is a topical ointment developed by the German company MEDIgene AG, indicated for the treatment of genital warts caused by human papillomavirus (HPV).

The medicinal components and clinical applications of tea polyphenols are well-documented in both domestic and international literature. Epidemiological surveys, clinical studies, and in vitro cell cultures have confirmed that tea polyphenols offer numerous health benefits, including antioxidant, anti-inflammatory, antitumor, anti-aging, and anti-radiation pharmacological effects [1].

In terms of toxicology, Isbrucker et al. [2] conducted animal experiments to analyze the toxicity of epigallocatechin gallate (EGCG) from the perspectives of genotoxicity and developmental toxicity. The results showed no genotoxic effects in mouse body fluid analyses, and no teratogenic or developmental toxicity was observed, indicating the high safety profile of tea polyphenols.

In studies on rheumatoid arthritis and osteoarthritis, tea polyphenols have been found to exert anti-inflammatory effects by inhibiting lipid peroxidation, regulating the transcription factor NF- κ B, and limiting the expression of inducible nitric oxide synthase. These mechanisms reduce the release of inflammatory factors such as TNF- α , IL-1, IL-6, and IL-8. In the context of osteoporosis, as early as 2003, Choi et al. [3] first reported that active components of tea polyphenols could promote the survival and alkaline phosphatase (ALP) activity of MC3T3-E1 osteoblasts by reducing TNF- α and IL-6, thereby inhibiting osteoblast apoptosis. Subsequently, Takai et al. [4] discovered in MC3T3-E1 osteoblast studies that EGCG inhibits platelet-derived growth factor-BB via the SAPK/JNK pathway, reducing the expression of IL-6 and IL-6 mRNA. In a recent study, Shen et al. [5] observed that green tea polyphenols may protect bone microstructure and strength by inhibiting inflammatory factors in rats.

Recently, Joo et al. [6] also found in their study on the effects of EGCG on bone marrow macrophages that EGCG may prevent LPS-mediated pro-inflammatory gene expression by blocking the NF- κ B and MAPK signaling pathways.

Thus, tea polyphenols can effectively inhibit inflammatory factors such as TNF- α and IL-6, suppress osteoblast apoptosis, and promote their growth. The inhibition of LPS may be a key mechanism through which tea polyphenols exert these effects.
[1] Qiao J, Kong X, Kong A, et al. Pharmacokinetics andbiotransformation of tea polyphenols. Curr Drug Metab, 2014, 15(1): 30-36.
[2] Isbrucker RA, Bausch J, Edwards JA, et al. Safety studies onepigallocatechin gallate (EGCG) preparations. Part 1:genotoxicity. Food and Chemical Toxicology, 2006,44(5): 626-635.
[3] Choi EM, Hwang JK. Effects of (+)-catechin on the function of osteoblastic cells. Biol Pharm Bull,2003,26(4): 523-526.
[4] Takai S, Matsushima-Nishiwaki R, Adachi S, et al. (-)-Epigallocatechin gallate reduces platelet-derived growth factor-BB-stimulated interleukin-6 synthesis in osteoblasts: suppressionof SAPK/JNK. Mediators Inflamm, 2008, DOI: 10.1155/2008/291808.
[5] Shen CL, Yeh JK, Samathanam C, et al. Green tea polyphenolsattenuate deterioration of bone microarchitecture in female ratswith systemic chronic inflammation. Osteoporosis International,2011,22(1): 327-337.
[6] Joo SY, Song YA, Park YL, et al. Epigallocatechin-3-gallateinhibits LPS-induced NF- K B and MAPK signaling pathways inbone marrow-derived macrophages. Gut Liver, 2012, 6(2): 188-196.

In practical application, the present invention provides a technical formulation that enables the active absorption of amino acids by the human reproductive system. This solution is applied to the sacral region of the human body. The sacrum and sacral nerves are critically important core areas for regeneration, possessing the functions of storing and continuously releasing electrical signals. The fixed pathway from the external genitalia to the sacral nerve plexus facilitates the regenerative repair of the motor-related nervous system and delays aging. When the solution is applied to the mucous membranes and skin of the external genitalia, such as through immersion for approximately half an hour, the sacral nerve plexus can transmit bioelectrical signals externally for about 24 hours. The bioelectrical signals transmitted by the sacral nerve plexus occur at a frequency of approximately 0.6 - 1 second per cycle. The solution can be applied to the labia, clitoris, urethral opening, vaginal orifice mucosa, and surrounding skin in females, and to the penis, glans penis, urethral opening, coronal sulcus mucosa, and surrounding skin in males. The sacral nerve plexus transmits bioelectrical signals along fixed pathways: 1. From the external genitalia to the sacral nerve plexus, then to the lumbar nerve plexus; 2. From the sacral nerve plexus to the lumbar nerve plexus, then to the cervical nerve plexus, medulla oblongata, cerebellum, occipital visual nerve area, and eyes; 3. From the sacral nerve plexus and lumbar nerve plexus to the sciatic nerve, tibial nerve, and common peroneal nerve, then to the medial and lateral plantar nerve areas; 4. From the sacral nerve plexus to the superior gluteal nerve and inferior gluteal nerve; 5. From the sacral nerve plexus to the pudendal nerve, reproductive system nerves, urinary system nerves, and defecation system nerves, delivering bioelectrical stimulation signals. The bioelectrical signals of the sacral nerve plexus can block or replace inflammatory pain signals, alleviating pain in the spine and spinal cord, reproductive system organs (such as pain in male and female genitalia), and male scrotal pain. They also inhibit motor neuron diseases, inflammation of the spine, and nerve damage in the sciatic, femoral, common peroneal, and gluteal nerves. Additionally, they suppress female reproductive system inflammation while restoring female reproductive capacity, address issues like erectile dysfunction and premature ejaculation, improve sperm count and quality in males, enhance penile rigidity and prolong pleasurable sensations, and restore genital sensitivity. The solution also inhibits bacterial and viral diseases in the genital organs, mitigates urinary system conditions such as uremia and kidney inflammation, prevents kidney atrophy, and reduces inflammation in internal organs like the liver, lungs, and pancreas. The sacral nerve plexus exhibits strong regenerative capabilities, contributing to the repair and regeneration of the entire human motor system, as well as internal organs such as the liver, pancreas, kidneys, and the urinary, reproductive, and defecation systems.

The immersion time does not necessarily need to be half an hour. For first-time users, after one week of immersion for half an hour to an hour, if no serious health issues are present, the daily immersion time can be reduced to 1 - 2 minutes, similar to the time required for eye drops, mouth rinsing, or intestinal cleansing. According to the sodium ion spiral curve, just 1 - 2 minutes of daily application can sustain the continuous regenerative repair process. The power of consistency should not be underestimated, and for the average person, one minute per day is sufficient.

So far, more than ten fixed pathways for signal transimition have been discovered through experiments: from the eyes to the heart; from the external genitalia to the sacral nerve plexus; from the sacral nerve plexus to the sciatic nerve and then to the plantar nerves; from the sacral nerve plexus to the lumbar plexus, then to the occipital bone, and finally to the eyes; from the sacral nerve plexus to the femoral nerve and then to the pudendal nerve; from the sacral nerve plexus to the superior and inferior gluteal nerves; from the nipples to the breasts, then to the cervical nerve plexus, and finally to the radial and ulnar nerves; from the nipples to the facial nerve; from the rectum to the sacral nerve plexus; from the oral cavity to the teeth and then to the neck nerves; from the nasal side to the trigeminal nerve and then to the area behind the ears. Additionally, there are pathways from the posterior auricular fold region to the forehead, joint areas, and others.

After extensive testing and experimentation, it has been observed that neurodegenerative diseases related to reproductive capacity are closely linked to aging. It is hypothesized that once the reproductive nerve regions of the human nervous system begin to degenerate, it leads to systemic neurodegenerative diseases, muscle degeneration, inflammation, tumors, and the exacerbation of chronic conditions, ultimately resulting in aging. This hypothesis aligns with the process of natural selection and evolution, as the decline and death of any animal often occur after the loss of reproductive capacity.

The formula solution can be absorbed by the nerves of the human reproductive system, rapidly restoring stable electrical signal transmission and activating the regenerative capacity of the sacral region. By reinstating the bioelectrical information characteristic of youth and reproductive periods, the human body has the potential to rejuvenate. Eye drops are also crucial, as they help maintain the youthfulness and health of the heart, brain, spine, and eyes.

In terms of pain suppression, the human body absorbs the formula solution and converts it into neurotransmitters, generating stable and continuous electrical signals that block the transmission of pain, numbness, itching, and other discomfort signals.

For patients with amyotrophic lateral sclerosis (ALS) and spinal cord injuries, immersing the external genitalia in the formula solution can activate the regenerative and reparative capabilities of the sacral nerve plexus, aiding in the repair of spinal and motor nerves. For patients with Alzheimer's disease and other brain disorders, methods such as eye drops and immersion of the external genitalia in the formula solution can help regenerate and repair neuronal damage in the brain.

For burn patients, the formula solution can be used for debridement and pain relief, avoiding the severe pain associated with manual debridement, while promoting repair and regeneration. This approach significantly reduces suffering.

The principle of regeneration is similar to that of octopus regeneration. When human neurotransmitters are in the correct state, and the extracellular sodium ion concentration around human nerve cells reaches or exceeds 440 mmol/L, tissue damage can be regenerated and repaired. After the human body absorbs the formula solution, the extracellular sodium ion concentration around nerve cells increases from 150 mmol/L to 4524 mmol/L and then decreases back to 150 mmol/L. It then rises again to 4524 mmol/L, and with the time coordinate, this sodium ion value exhibits a stable spiral curve. According to the natural selection principle of survival of the fittest, when the extracellular sodium ion concentration around nerve cells reaches 4524 mmol/L, it can eliminate and eradicate malignant tumors and inflammatory tissues. The competition of sodium ion values aligns with evolutionary theory.

In practical application, the present invention provides a technical formulation that enables the automatic absorption of amino acids by the human reproductive system. The formula solution is applied to the female reproductive system, breasts, and male nipple areas. By immersing or applying the formula solution to the nipples for approximately half an hour daily, the nipple area actively absorbs the solution, generating bioelectrical signals that propagate along fixed pathways. Pathway Six: Bioelectrical signals travel from the nipples to the brachial nerve plexus and the tibial nerve plexus, then to the ulnar nerve, inhibiting inflammation and pain in the arms, chest, and back. Pathway Seven: Bioelectrical signals travel from the nipples to the cervical nerve plexus and then to the facial nerve, inhibiting lymphatic tumors around the neck; suppressing facial sagging by gradually increasing muscles such as the platysma, sternocleidomastoid, trapezius, and masseter; inhibiting mastitis, fibroadenomas, mammary hyperplasia, and breast fluid accumulation; suppressing breast tumors and cancer, as well as pain in the breasts, neck, and arms. Breast lumps and hard masses will soften, and breast fluid accumulation will rapidly decrease. The solution also inhibits neck masses and tumors, prevents degeneration of neck muscles and sagging of facial muscles, and reduces the formation of neck wrinkles and wrinkles around the ears.

There are numerous types of cancer, and the number of deaths is significant. However, cancer has a commonality: elevated sodium ion and amino acid concentrations. For example, the sodium ion concentration in the liquid environment of malignant tumors is 451 mmol/L, which is three times the extracellular sodium ion concentration of ordinary human nerve cells (150 mmol/L). Cancer cells metastasize freely in the human body, but when the formula solution enters the human nervous system, the extracellular sodium ion concentration around nerve cells can reach as high as 4524 mmol/L, which is ten times that of malignant tumors. The concentration of 4524 mmol/L is extremely safe for humans, making it relatively easy to suppress malignant tumors with this level of extracellular sodium ions around nerve cells. Additionally, this concentration can rapidly inhibit pain and inflammation. Currently, breast cancer causes significant harm to women. According to the IARC World Health Organization 2020 statistics, there were 2.26 million cases of breast cancer, surpassing the 2.2 million cases of lung cancer. Although our current sample size is limited, the effects are rapid, the process is painless, and the treatment is non-invasive, making it an innovative therapy for malignant tumors such as breast cancer. This method also quickly restores flexibility to the shoulder and neck areas and facilitates rapid regeneration and repair of the nervous system. The regeneration and repair of the nervous and immune systems will lead to the restoration of the blood and muscular systems, ultimately resulting in skeletal regeneration and repair.

In practical application, the present invention provides a technical formulation for the automatic absorption of amino acids by the human reproductive system. The formulation is either (a1) or (a2):
(a1) A combination of sodium chloride and tea components, where the tea components are any combination of commonly used teas;
(a2) A combination of sodium chloride and Tieguanyin oolong tea.

Teas can be classified in various ways, such as by fermentation degree. Generally, teas are divided into six major categories: oolong tea with a fermentation degree of 15% - 30%, green tea with 0% fermentation, dark tea with 100% fermentation, white tea with 5% - 10% fermentation, yellow tea with 10% - 20% fermentation, and black tea with 70% - 90% fermentation. The components of different teas are relatively similar, especially the more than 20 types of amino acids. In experiments, teas with longer fermentation times showed poorer efficacy, while oolong tea and green tea demonstrated better and more stable results. Among them, Tieguanyin oolong tea has shown high stability over years of testing.

In practical application, the present invention also provides a method for preparing the aforementioned technical formulation for the automatic absorption of amino acids by the human reproductive system. The method is characterized by extracting the tea components of the formulation through physical means and then mixing them with sodium chloride to synthesize the formula solution.

Human regenerative function requires stable neurotransmitters. One example of the formula solution is as follows: sodium chloride reaches the maximum solubility in water (≈26.47%), and the tea components exceed a concentration of 40 mg/ml. The higher the concentration of tea components in the saturated formula solution, the higher the absorption efficiency of the human nervous system, the stronger the capacity for neural repair and regeneration, and the better the effects of pain and inflammation suppression.

From the perspective of sodium chloride reaching saturation concentration, the present invention develops methods to inhibit disease invasion. In the described formulation, sodium chloride reaches the maximum solubility in water (≈26.47%). Extensive human eye drop experiments have confirmed that the combination of saturated sodium ions and high-concentration tea components can safely circulate in the human body, benefiting mitochondrial regeneration, repair, and metabolism, muscle growth, and neural damage repair. Under the correct specific conditions of neurotransmitters, using the highest concentration of sodium ions in medicine can bring the following benefits: facilitating fixed patterns and outcomes in medical practice; aiding in the screening of truly effective central nervous system drugs and eliminating toxic ones; significantly alleviating disease-related suffering and shortening treatment duration.

The purpose of physical methods is to maintain the integrity of the components. With the participation of saturated sodium chloride, the human nervous system actively absorbs over 500 components, which are then converted into neurotransmitters with diverse types and relatively stable proportional relationships.

We have conducted numerous purification experiments, such as the atomization extraction method. Through atomization, we collected the liquid after atomization. Tests before and after atomization revealed that the pre-atomization liquid was golden in color, while the post-atomization liquid was light yellow and nearly transparent. After eye drop tests, the formula solution made from pre-atomization components performed significantly better than the post-atomization solution. This demonstrates that certain components were reduced after atomization, thereby decreasing the efficiency of the nervous system. Thus, maintaining a high concentration of the formula solution is beneficial for preserving effective treatment outcomes.

The differences among various tea components are minimal. For instance, it has been found that tea contains over 20 types of amino acids. Different teas exhibit slight variations in amino acid types, tea polyphenol content, and inorganic substance types. The main components of tea include: Amino acids: theanine, asparagine, glutamic acid, glycine, serine, tryptophan, valine, histidine, arginine, threonine, alanine, proline, cysteine, tyrosine, methionine, lysine, isoleucine, leucine, phenylalanine, etc. ; Vitamins: C, B1, B2, B3, B5, B6, B9 (folic acid), B12, P1, H (biotin), A, D, E, K, etc.; Alkaloids: caffeine, theophylline, theobromine, etc. ; Tea polyphenols: catechins, flavonoids, anthocyanins, phenolic acids, etc.; Fatty acids and lipids: saturated fatty acids, monounsaturated fatty acids, polyunsaturated fatty acids, phospholipids, glycerides, sulfur lipids, glycolipids, etc. ; Inorganic substances: potassium, phosphorus, calcium, magnesium, iron, sulfur, aluminum, fluorine, etc.

Research on human neurotransmitters is highly challenging. Based on the inferences of biologists, such as the content in Yan Ning's lectures, the number of human neurotransmitter types is estimated to be around 200, while some scientists believe it could be as high as 1,000. Experiments on the correspondence between tea components combined with sodium chloride and human neurotransmitters are still ongoing. Through extensive foundational experiments, we believe that the combination of tea and sodium chloride is safe for human neurotransmitters. This is because tea contains a comprehensive range of amino acids, a wide variety of vitamins, and abundant types of fatty acids, lipids, and inorganic substances, all of which are essential for neurotransmitters. Thus, we speculate that approximately three-quarters of the components in tea, or around 400 types, participate in the active absorption process of the nervous system and are converted into over 400 neurotransmitters. However, this remains a hypothesis in foundational research and requires extensive work and time to refine.

In practical application, the present invention provides a technical formulation for the automatic absorption of amino acids by the human reproductive system. The formula solution can be applied to any part of the human body. It targets human sodium and chloride ion channels, and the nervous system actively absorbs the solution, converting it into stable human neurotransmitters. This enables the regenerative and reparative capabilities of the nervous and immune systems, subsequently leading to the regeneration and repair of tissues and organs such as the blood system, muscles, and bones. When neurotransmitters in the human body are relatively stable, changes in sodium levels serve as a critical means of biological competition. For example, in the context of the formula, the sodium ion concentration in the fluid environment of malignant cancer cells is 451 mmol/L, while in inflammatory environments, it ranges from 200 to 300 mmol/L. In contrast, the extracellular sodium ion concentration around octopus and squid nerve cells is 440 mmol/L, which is an important reference value for animal regeneration. When the extracellular sodium ion concentration around human nerve cells rises from 150 mmol/L to 4524 mmol/L and then falls back to 150 mmol/L, the peak sodium ion concentration in humans is approximately ten times that of octopuses, ten times that of malignant tumors, and twenty times that of inflammatory environments. The increase from 150 mmol/L to 4524 mmol/L represents a 30-fold rise, significantly enhancing the strength of bioelectrical stimulation signals. As a result, the human body improves its ability to suppress inflammation and cancer cells by 1 to 30 times. This spiral curve process facilitates the regeneration and repair of the nervous system. The nervous system has dozens of tentacle-like ducts inserted into the immune system, with which it is closely interconnected. The bioelectrical signals of the nervous system rapidly enter the immune system, thereby increasing the bioelectrical signals received by the immune system by 1 to 30 times. Consequently, the immune system's ability to secrete mucus is greatly enhanced, as is its capacity to generate immune cells. The production and maturation of immune cells within the immune system accelerate, and the speed at which inflammatory substances are broken down, eliminated, and transported out of the body significantly increases. This improves the body's ability to stop bleeding, debride wounds, and detoxify, while also suppressing pain and itching, inhibiting tissue fibrosis, and preventing tumors and vascular malformations. The formula serves as a supplement for human amino acids and neurotransmitters.

The fluctuating process of sodium levels involves not only rapid responses from the nervous system but also nearly simultaneous reactions from the immune system. In May 2022, Professor Wei from Shanghai University conducted experiments demonstrating that mice expel viruses invading the brain through the meningeal lymphatic vessels to the cervical lymph nodes, revealing a new pathway for viruses to spread from the central nervous system to the peripheral system. In 2021, researchers at the Feinstein Institute for Medical Research in the United States discovered complex communication between two intricate biological systems-the nervous system and the immune system. They also found that activating certain neurons enhances antibody production. A surprising and unexpected finding from this new research is that the immune system response is controlled by neurons responsible for transmitting pain signals.

For a long time, the nervous system and immune system of the body were considered independent systems. However, recent research has revealed direct cellular interactions between the two. This new study, published in *Cell*, highlights the mediated communication between the immune system and the nervous system. Researchers discovered that 20 neurons innervate a single lymph node.

Scientists have also found that this communication is bidirectional: neurons themselves can regulate cells within the lymph nodes. This discovery confirms that pain-sensing neurons can broadly alter the local environment of lymph nodes.

Based on the above literature, it can be confirmed that the nervous system controls the immune system. Extensive experiments using the formula solution have demonstrated the simultaneous action of the nervous and immune systems. For example, mucus clearance in the lungs can reach 60 ml within half an hour; mucus secretion after eye drops, nasal drops, and intestinal clearance are also observed. When the formula solution is applied to the nipples, a rapid reduction in breast fluid accumulation is noted in patients, suggesting drainage via the lymphatic system. Similarly, when the formula solution is applied to the external genitalia, inflammatory components in the kidneys, liver, pancreas, bladder, and cervix are rapidly cleared. A sodium ion concentration of 4524 mmol/L enhances almost all bodily functions, such as rapid hemostasis, itch relief, and swift suppression of pain from burns.

According to the literature, multiple studies have confirmed that changes in cell membrane potential are associated with the activity of cell mitosis. An increase in the concentration of cations within cells indicates a higher likelihood of malignancy. The percentage of Na⁺ concentration shows a gradually increasing gradient in normal tissues, benign or borderline tumors, and malignant tumor cells, at 0.05%, 0.05% - 0.07%, and 0.15% - 0.2%, respectively. The sodium ion concentration in tumor and peritumoral tissues is 50% - 60% higher than in normal tissues. The assessment of tumor properties based on Na⁺ concentration has long been recognized as one of the effective methods. As early as 1922, German biologists discovered that the electrolyte composition of malignant tumors differs from that of normal biological cells, with a significant increase in alkaline ion concentration. In the 1980s, scientists found that the degree of increase in intracellular alkaline ion concentration is positively correlated with the rate of cell division, regardless of cell type. Oncologists from the United States, Canada, Switzerland, Hungary, and other countries have reached the same conclusion in their research: an increase in intracellular alkaline ions indicates a higher probability of malignancy. According to measurements by J. Cameron, the sodium ion concentration in ordinary extracellular fluid is 138 - 145 millimoles per kilogram (mM), while in malignant tumor extracellular fluid, it is 451 mM. This value of 451 is very close to the sodium ion concentration outside the nerve cell membranes of octopuses, which is 440 mM.

Fibrosis refers to the pathological process in which inflammation leads to the necrosis of parenchymal cells in organs, resulting in abnormal increase and excessive deposition of extracellular matrix within the tissue. Mild cases involve fibrosis, while severe cases cause structural damage and sclerosis. Tissue fibrosis is a major cause of disability and death in many diseases, commonly occurring in the lungs, cardiovascular system, liver, pancreas, kidneys, spleen, eyes, nervous system, and other organs.

Inhibiting fibrosis primarily targets sodium channels. It is hypothesized that the range of 440 - 451 millimoles per liter is a critical numerical region for regeneration, as organ fibrosis is rarely observed in octopuses in nature. When the extracellular sodium ion concentration of human nerve cells persistently and chronically increases from 150 millimoles per liter to 440 millimoles per liter, sustained repair and regeneration of tissues and organs occur.

In the experiment on tail regeneration in turtles, a combination of saturated sodium chloride and longan leaves was used. During the months of regeneration, the black scab on the turtle's stomach slowly grew and advanced, and the newly regenerated muscles, bones, and skin showed no signs of fibrosis. Similarly, in the case of an infected cavity in a human calf, regenerative fluid and white stem cells were found beneath the black scab. The entire repair process avoided fibrosis, and the newly regenerated skin was smooth and lacked texture initially. However, over time, the skin texture gradually repaired, indicating that the regenerative repair process is continuous and progressively breaks down inflammatory fibrosis. In the case of breast lumps, the formation of the lumps had persisted for over a decade, accompanied by inflammatory fluid accumulation and an increase in the size and number of hard masses. After several weeks of immersing the nipples in the formula solution, the fluid gradually disappeared, and the hard masses progressively broke down, consistent with the sodium ion spiral curve process, gradually decomposing inflammatory fibrosis. Several participants with lung nodules, thyroid nodules, and calcified tissues from lumbar disc herniation experienced a gradual reduction in lung and thyroid nodules and the dissolution of calcified tissues in the lumbar disc over several months of using the formula. Such cases are increasingly observed, and the analysis suggests that changes in sodium ion concentration drive the alterations in nodules. The sodium ion concentration in the extracellular fluid of inflammatory regions is 200 - 300 mmol/L, while in malignant tumor regions, it is 451 mmol/L. Thus, it can be inferred that the sodium ion concentration in the physical environment of fibrosis falls within the range of 200 - 451 mmol/L. When the sodium ion concentration exceeds this range and reaches 4524 mmol/L, the stable environment of fibrosis is disrupted, and fibrotic nodules caused by inflammation automatically break down.

The repair and regeneration of the nervous system inevitably depend on the repair and regeneration of mitochondria. Literature studies indicate that sodium ions on mitochondria can regulate the fluidity of cell membranes. However, high salt levels can lead to mitochondrial dysfunction, as sodium ions enter the mitochondria and subsequently inhibit the electron transport chain located on the inner mitochondrial membrane, reducing ATP production. When high salt acts on the nervous system, it can cause severe functional issues in a large number of mitochondria. Through experiments such as the eye drop tests mentioned above, mitochondrial regeneration and repair can also be achieved under high salt conditions. The formula solution can be absorbed by the nervous system and converted into "correct" neurotransmitters, which are highly compatible with the body's original neurotransmitters. As a result, under high salt conditions, saturated sodium and chloride ions do not damage mitochondria but instead facilitate their extensive regeneration and healthy metabolism. The exact reasons for the extensive regeneration and healthy metabolism of mitochondria under these conditions are not yet fully understood. It is hypothesized that the approximately 500 components in the formula solution are absorbed, with most types converted into neurotransmitters that are diverse and maintain stable proportions. When the neurotransmitter substrates are "correct" and sufficiently abundant, sodium ions do not inhibit the electron transport on the inner mitochondrial membrane and thus do not reduce ATP production. With sufficient neurotransmitter substrates, sodium and chloride ions function correctly and healthily. Additionally, mitochondrial chloride channels play important roles in regulating angiogenesis in endothelial cells, cell cycle, and cell differentiation. They are widely involved in regulating mitochondrial matrix pH, mitochondrial volume, and pathophysiological responses such as cell death, particularly exerting protective effects in heart diseases.

In practical application, the present invention provides the use of the aforementioned technical formulation for the automatic absorption of amino acids by the human reproductive system in the preparation of drugs for preventing or treating human diseases. The efficacy of the drug is as described above. The dosage form of the drug is any one of sprays, injectable preparations, tablets, capsules, granules, suspensions, and pills, and it further includes pharmaceutically acceptable carriers and excipients. In practical applications, the methods of administering the formulation of the present invention to the entire human body include but are not limited to injections, topical application, instillation, nebulization, immersion, patches, etc. The formulation can be produced in forms such as tablets, injections, nebulizers, ointments, patches, topical applications, acupoint patches, aerosol sprays, bottled liquids, capsules, and any other pharmaceutical manufacturing processes or forms, either individually or in combination.

The formulation drug is a natural product but exhibits very high stability. It does not require preservatives or stabilizers, is easy to produce, and can be stored for several months after preparation. However, researchers replicating the experiments are reminded that in regeneration experiments, it is best to use the formula solution stored for about one week, as this yields the optimal effect. If stored for more than two months, the efficacy of the formulation decreases by approximately 30%.

Any person skilled in the art, within the technical scope disclosed by the present invention, may easily envision modifications, variations, or substitutions of the formulation, all of which should fall within the protection scope of the present invention. Therefore, the protection scope of the invention should be defined by the claims.

## Claims

1. A technical formulation that enables the active absorption of amino acids by the human reproductive system, wherein, in a sacral region of the human body, sacrum and sacral nerves are critically important core areas for regeneration, possessing the functions of storing and continuously releasing electrical signals; fixed pathways from the external genitalia to the sacral nerve plexus facilitate the regenerative repair of the motor-related nervous system and delays aging; when the solution is applied to the mucous membranes and skin of the external genitalia, such as through immersion for approximately half an hour, the sacral nerve plexus can transmit bioelectrical signals externally for about 24 hours; the bioelectrical signals transmitted by the sacral nerve plexus occur at a frequency of approximately 0.6 - 1 second per cycle; the solution can be applied to the labia, clitoris, urethral opening, vaginal orifice mucosa, and surrounding skin in females, and to the penis, glans penis, urethral opening, coronal sulcus mucosa, and surrounding skin in males; the sacral nerve plexus transmits bioelectrical signals along fixed pathways: path one, from the external genitalia to the sacral nerve plexus, then to the lumbar nerve plexus; path two, from the sacral nerve plexus to the lumbar nerve plexus, then to the cervical nerve plexus, medulla oblongata, cerebellum, occipital visual nerve area, and eyes; path three, from the sacral nerve plexus and lumbar nerve plexus to the sciatic nerve, tibial nerve, and common peroneal nerve, then to the medial and lateral plantar nerve areas; path four, from the sacral nerve plexus to the superior gluteal nerve and inferior gluteal nerve; path five, from the sacral nerve plexus to the pudendal nerve, reproductive system nerves, urinary system nerves, and defecation system nerves, delivering bioelectrical stimulation signals; the bioelectrical signals of the sacral nerve plexus can block or replace inflammatory pain signals, alleviating pain in the spine and spinal cord, reproductive system organs (such as pain in male and female genitalia), and male scrotal pain; they also inhibit motor neuron diseases, inflammation of the spine, and nerve damage in the sciatic, femoral, common peroneal, and gluteal nerves.; additionally, they suppress female reproductive system inflammation while restoring female reproductive capacity, address issues like erectile dysfunction and premature ejaculation, improve sperm count and quality in males, enhance penile rigidity and prolong pleasurable sensations, and restore genital sensitivity; the solution also inhibits bacterial and viral diseases in the genital organs, mitigates urinary system conditions such as uremia and kidney inflammation, prevents kidney atrophy, and reduces inflammation in internal organs like the liver, lungs, and pancreas; the sacral nerve plexus exhibits strong regenerative capabilities, contributing to the repair and regeneration of the entire human motor system, as well as internal organs such as the liver, pancreas, kidneys, and the urinary, reproductive, and defecation systems.

2. A technical formulation that enables the automatic absorption of amino acids by the human reproductive system, wherein, the formula solution is applied to the female reproductive system, breasts, and male nipple areas; by immersing or applying the formula solution to the nipples for approximately half an hour daily, the nipple area actively absorbs the solution, generating bioelectrical signals that propagate along fixed pathways; pathway six: bioelectrical signals travel from the nipples to the brachial nerve plexus and the tibial nerve plexus, then to the ulnar nerve, inhibiting inflammation and pain in the arms, chest, and back; pathway seven: bioelectrical signals travel from the nipples to the cervical nerve plexus and then to the facial nerve, inhibiting lymphatic tumors around the neck; suppressing facial sagging by gradually increasing muscles such as the platysma, sternocleidomastoid, trapezius, and masseter; inhibiting mastitis, fibroadenomas, mammary hyperplasia, and breast fluid accumulation; suppressing breast tumors and cancer, as well as pain in the breasts, neck, and arms; breast lumps and hard masses will soften, and breast fluid accumulation will rapidly decrease; the solution also inhibits neck masses and tumors, prevents degeneration of neck muscles and sagging of facial muscles, and reduces the formation of neck wrinkles and wrinkles around the ears.

3. A technical formulation for the automatic absorption of amino acids by the human reproductive system, wherein, the formulation is either (a1) or (a2):
(a1) A combination of sodium chloride and tea components, where the tea components are any combination of commonly used teas;
(a2) A combination of sodium chloride and Tieguanyin oolong tea.

4. The method for preparing the formulation according to any one of claims 1-3, **characterized in that** tea components of the formulation are extracted by physical methods and mixed with sodium chloride for synthesis.

5. The formulation according to any one of claims 1-4, **characterized in that**, the formula solution can be applied to any part of the human body; it targets human sodium and chloride ion channels, and the nervous system actively absorbs the solution, converting it into stable human neurotransmitters; this enables the regenerative and reparative capabilities of the nervous and immune systems, subsequently leading to the regeneration and repair of tissues and organs such as the blood system, muscles, and bones; when neurotransmitters in the human body are relatively stable, changes in sodium levels serve as a critical means of biological competition; for example, in the context of the formula, the sodium ion concentration in the fluid environment of malignant cancer cells is 451 mmol/L, while in inflammatory environments, it ranges from 200 to 300 mmol/L; in contrast, the extracellular sodium ion concentration around octopus and squid nerve cells is 440 mmol/L, which is an important reference value for animal regeneration; when the extracellular sodium ion concentration around human nerve cells rises from 150 mmol/L to 4524 mmol/L and then falls back to 150 mmol/L, the peak sodium ion concentration in humans is approximately ten times that of octopuses, ten times that of malignant tumors, and twenty times that of inflammatory environments; the increase from 150 mmol/L to 4524 mmol/L represents a 30-fold rise, significantly enhancing the strength of bioelectrical stimulation signals; as a result, the human body improves its ability to suppress inflammation and cancer cells by 1 to 30 times; this spiral curve process facilitates the regeneration and repair of the nervous system; the nervous system has dozens of tentacle-like ducts inserted into the immune system, with which it is closely interconnected; the bioelectrical signals of the nervous system rapidly enter the immune system, thereby increasing the bioelectrical signals received by the immune system by 1 to 30 times; consequently, the immune system's ability to secrete mucus is greatly enhanced, as is its capacity to generate immune cells; the production and maturation of immune cells within the immune system accelerate, and the speed at which inflammatory substances are broken down, eliminated, and transported out of the body significantly increases; this improves the body's ability to stop bleeding, debride wounds, and detoxify, while also suppressing pain and itching, inhibiting tissue fibrosis, and preventing tumors and vascular malformations; the formula serves as a supplement for human amino acids and neurotransmitters.

6. The formulation according to any one of claims 1-5, **characterized in that** it is used in the preparation of a drug for preventing or treating human diseases; the efficacy of said drug includes the content described in any one of claims 1-5; the dosage form of said drug is any one of spray, injectable preparation, tablet, capsule, granule, suspension, and pill, and further comprises pharmaceutically acceptable carriers and excipients; in practical applications, the formulation of the present invention can be applied to the entire human body in methods including but not limited to injections, topical application, instillation, nebulization, immersion, patches, etc; the finished product forms that can be produced include tablets, injections, nebulizers, ointments, patches, topical applications, acupoint patches, nebulized sprays, bottled liquids, capsules, and any other forms or combinations involved in pharmaceutical manufacturing processes.

## Amended claims

### Amended claims under Art. 19.1 PCT

1. A technical formulation for the automatic absorption of amino acids by the human reproductive system, wherein, the formulation is either (a1) or (a2):
(a1) A combination of sodium chloride and tea components, where the tea components are any combination of commonly used teas;
(a2) A combination of sodium chloride and Tieguanyin oolong tea.

2. The formulation according to claim 1, **characterized in that** the preparation method of the formulation involves extracting the tea components of the formulation through physical methods, where the tea is boiled in water to release its components, and then combined with sodium chloride for synthesis.

3. The use of the composition of the formulation according to claim 1 or 2 in the preparation of drugs for preventing and treating nervous system diseases and immune system diseases, as the concentration of the formulation solution increases, the inhibitory effect on diseases enhances; the route of administration involves absorption through the skin and mucous membranes by the nervous system, targeting human sodium ion channels and chloride ion channels; the nervous system actively absorbs the formulation solution, supplements human neurotransmitters, and stably and continuously enhances and activates electrical signals; due to the enhanced electrical signals in the nervous system, the immune system responds rapidly and directly, expelling or transferring inflammatory substances; in particular, eye drops, lung nebulization, oral and rectal applications result in the expulsion of large amounts of inflammatory mucus, quickly suppressing inflammation, eliminating swelling and pain, and achieving repair of the nervous and immune systems; this gradually repairs blood vessels, muscles, bones, and other tissues and organs; the electrical signals generated by the application of the formulation solution to the eyes reach the occipital lobe of the brain through the nervous system, extend to the sacral nerves, and reach the heart, inhibiting inflammation and pain in the eyes, brain, spinal cord, and heart; application of the formulation solution to the lungs can expel phlegm and relieve coughs, suppress lung inflammation, and inhibit pulmonary fibrosis; application to the rectum can expel inflammatory mucus, suppress perianal inflammation and pain, intestinal inflammation, and relieve constipation; application to the nasal cavity can inhibit rhinitis, trigeminal nerve inflammation, and pain; application to the oral cavity can inhibit dental inflammation and pain, gum and mucosal inflammation and pain, and prevent gum muscle atrophy; it also suppresses internal organ inflammation and tumors, alleviates pain and itching, and accelerates hemostasis and wound debridement.

4. The use of the composition of the formulation according to claim 1 or 2 in the preparation of drugs for preventing and treating reproductive system diseases, application of the formulation solution to male and female reproductive organs can suppress reproductive system inflammation, alleviate pain, enhance female reproductive capacity, increase sperm count and quality in males, restore hardness, repair genital sensitivity, and inhibit erectile dysfunction and premature ejaculation; application of the formulation solution to male and female nipples, when used consistently by females, has a breast-enhancing effect, suppresses breast inflammation and tumors, and inhibits inflammation and pain in the breasts, chest, back, neck, and arms, as well as muscle inflammation; continuous application of the formulation solution to the reproductive organs and nipples inhibits motor neuron diseases, spinal cord inflammation and pain, and inflammation and pain in the sciatic nerve, femoral nerve, common peroneal nerve, superior gluteal nerve, and leg nervous system.

5. The formulation according to claim 1 or 2, **characterized in that**, in practical applications, the methods of administering the formulation solution to the entire human body include injections, topical application, instillation, nebulization, immersion, and patches; the dosage forms of the drug include any of sprays, injectable preparations, tablets, capsules, granules, suspensions, and pills, and further comprise pharmaceutically acceptable carriers and excipients; the finished product forms that can be produced include tablets, injections, nebulizers, ointments, patches, topical applications, acupoint patches, nebulized sprays, bottled liquids, capsules, and any other forms or combinations involved in pharmaceutical manufacturing processes.
